# EUROPEAN PATENT SPECIFICATION

(11) **EP 1 320 767 B1**
(45) Date of publication and mention of the grant of the patent: **30.11.2005**
(21) Application number: 01969108.8
(22) Date of filing: 25.09.2001
(51) Int. Cl.: G01V 1/00

(54) **ACOUSTIC MONITORING OF CONCRETE VESSELS AND STRUCTURES**
AKUSTISCHE ÜBERWACHUNG VON BETONGEFÄSSEN UND BETONBAUWERKEN
SURVEILLANCE DE CUVES ET DE STRUCTURES EN BETON

(30) Priority: 25.09.2000 CA 2320631
(43) Date of publication of application: 25.06.2003
(73) Proprietor: PURE TECHNOLOGIES LTD., Calgary, Alberta, T2R 1L5 (CA)
(72) Inventor: PAULSON, Peter, O., Calgary, Alberta T2V 4M7 (CA)
(74) Representative: Chapman, Paul Nicholas
(86) International application number: PCT/CA2001/001341
(87) International publication number: WO 2002/025316

(56) References cited:
- DE-C- 852 771
- US-A- 4 918 993
- US-A- 5 798 457
- YUYAMA SHIGENORI ET AL: "Acoustic emission evaluation of structural integrity in repaired reinforced concrete beams" MATER EVAL;MATERIALS EVALUATION JAN 1994, vol. 52, no. 1, January 1994 (1994-01), pages 88-90, XP001058271
- KAWAHARA MASANORI ET AL: "AE measurements for evaluation of defects in FRP pressure vessels" PROCEEDINGS OF THE 1995 JOINT ASME/JSME PRESSURE VESSELS AND PIPING CONFERENCE;HONOLULU, HI, USA JUL 23-27 1995, vol. 297, 1995, pages 59-64, XP008000106 ASME Pressure Vessels Piping Div Publ PVP;American Society of Mechanical Engineers, Pressure Vessels and Piping Division (Publication) PVP; High Pressure Technology 1995 ASME, New York, NY, USA
- DATABASE WPI Derwent Publications Ltd., London, GB; AN 1984023096 XP002188169 & SU 1 006 679 A (EVDOKIMOV VIKTOR ;VOJNA LEONID S (SU); ELISEEVA TATYANA N (SU)) 23 March 1983 (1983-03-23)

## Description

The present invention relates to the monitoring of concrete vessels (such as concrete pipes or containment vessels including water towers and containment vessels for containing accidental discharge of radioactive gases at nuclear facilities) and concrete structures (such as buildings or bridges) to determine possible deterioration of their structural integrity.

### Background

Many concrete vessels and structures contain wire reinforcements. It is known to monitor such vessels and structures acoustically, to record the signals arising from the breaking of the wire reinforcements. Examples of such monitoring are shown in U.S.P. 5,798,457 (Paulson), issued August 25, 1998 and U.S.P.6,082,193 (Paulson), issued July 4, 2000.

Other than events caused by breaking of pre- or post-tensioned reinforcing wires, concrete vessels and structures normally do not emit significant acoustic events unless they are placed under stress.

It is well known that on loading of a concrete structure, small cracking sounds can be heard whenever the loading exceeds a previous maximum. This is called the Kaiser effect. A phenomenon which was originally disclosed in west german patent No. 852,771 to J. Kaiser. It is thought to occur because of the creation of microfractures in the concrete formed by the new level of loading.

Although many concrete vessels and structures have wire reinforcements, there are unreinforced structures, and structures with non-wire reinforcements, such as steel reinforcing bars. The Kaiser effect occurs in any concrete structures or vessels, whether they have wire reinforcement or not.

Systems which monitor structures or vessels for wire breakage do not seek to record the Kaiser effect, because there are other instruments, such as pressure or strain gauges, to record events which load structures or vessels. The other instruments give a quantitative determination of loading, and do not only determine loading beyond the previous maximum loading level.

It is noted, that S. Yuyama et al. ('Acoustic emission evaluation of structural integrity in repaired reinforced concrete beams', MATERIAL EVALUATION Jan. 1994, vol. 52, no. 1, pages 88-90) concluded, under different conditions though, that the Kaiser effect can be a good indicator for evaluation of structural integrity in repaired reinforced concrete beams.

Further, the acoustic events created by the Kaiser effect do not give off nearly as much energy as those created by wire breakage. Wire breakage typically gives an acoustic event of approximately the same magnitude as hitting the structure with a Schmidt hammer. In a recent test in which the Kaiser effect was caused in a reinforced concrete nuclear containment vessel, a Schmidt hammer gave an acoustic event yielding 30dB of energy, whereas the Kaiser effect gave 480 events having -30 to 0 dB and only 50 events over 0 dB, none of which were 30 dB or over. Further, Kaiser effect events occur in a narrow frequency band, whereas wire breaks give a much broader range of frequencies. Thus, arrays for listening for wire breaks typically either exclude Kaiser effect events because they are too small or do not have the correct frequency characteristics to be examined as likely wire breaks.

### The Invention

It has now been found that repeated or long term or comparison monitoring of small acoustic events, of the same general frequency and amplitude as those detected in the Kaiser effect, provides valuable information to determine structural damage occurring because of corrosion of reinforcing steel wires or bars, or damage from external forces such as earthquake or collision.

Typically, such events occur in one or more narrow bands of frequencies (specific to the container or structure being monitored) within the 2-12 KHz range, with most signals of interest being in the 8-12KHz range. It is usually sufficient to record signals in the frequency range of about 2KHz to 12KHz in order to get all signals of probable interest. They can be detected using any suitable acoustic detector coupled to the concrete structure so as to receive acoustic emissions at these frequencies. Acoustically, the noises are sharp cracking sounds, and can be likened to the sound of popcorn popping. If several acoustic detectors are used, the locations within the vessel wall or the structure at which the cracking sounds originate can often be located, by using methods analogous to those used to locate wire breaks in the two Paulson patents cited above.

The present invention comprises a method of inspection of concrete structures as set forth in the claims.

### Drawings

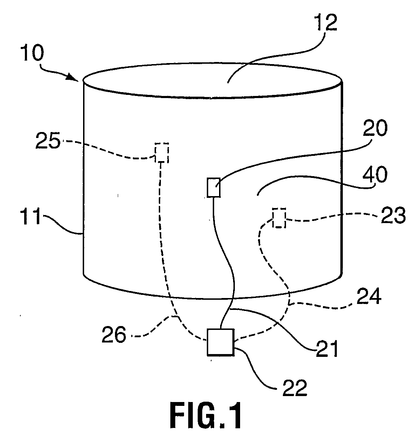

The present invention will be further explained with respect to the drawing, Figure 1, which is a perspective view (not to scale) of a concrete pressure vessel with a suitable monitoring equipment configuration for the invention.

### Periodic Monitoring Embodiment

According to one embodiment of the invention, a base test is made with the structure or vessel loaded to a predetermined amount. In the case of a vessel such as a water tank or nuclear containment vessel, the loading can conveniently be by pressurizing the contents of the vessel to a predetermined pressure in the case of a structure, such as a bridge or a building, the test can be by placirig test loads of predetermined weight in prechosen locations on the bridge deck or floor of the building. Loads of predetermined weight can also be used for vessel testing, although they are not preferred.

The loading for the base test (hereinafter called the "base load") should be chosen to be a loading which exceeds the loading that the vessel or structure would encounter in normal use. Typically, a base load which is 1.5 to 10 times the load encountered in normal use would be suitable. It is of course desirable that the load chosen is within the vessel's or structure's design limits, to prevent damage to the vessel or structure.

It is not necessary that the base load exceed any load which has previously been applied to the vessel or structure. If it does exceed the greatest previous load that has been applied, a Kaiser effect will occur when and where it is applied. This can be of interest if multiple sensors have been positioned, because the Kaiser effect arises from microcracking and often the locations of the microcracking can be found precisely, from the locations of the sensors and the time of arrival of the acoustic signal at each sensor, as disclosed in the Paulson patents cited above. These locations of microcracks are useful to know, as these locations are likely to be ones where there is considerable stress, and which would benefit from frequent inspection by conventional means during the life of the structure.

If the base load does not exceed any load previously applied to the vessel or structure, there will be no Kaiser effect. In some cases, previous loading may be exceeded in some portions of a structure or vessel but not in others, in which case there will be a Kaiser effect in the portions where previous loading was exceeded, and no Kaiser effect in other portions.

The presence or absence of the Kaiser effect when the base test is carried out is not important to the invention. What is important in the embodiment being described is that testing is done on several occasions spaced over time, using the base load or a somewhat lesser load which still exceeds the normal load encountered in use of the vessel or structure. Because the acoustic events propagate easily through concrete, it is not necessary that the sensors be in exactly the same position in each test, although it may be convenient to leave the sensors permanently in position between tests in most cases, to reduce the setup time for each test. The loads should preferably be applied at or near the location where they were applied in the base test, to facilitate comparison between test results. In the case of pressurizing a container, the load will automatically be applied in the same location as the base test if the same container, or the same subcompartment of a container, is pressurized. Where the load is a weight, applying the load at or near the previous location can conveniently be accomplished by marking the structure or vessel at the time of the base test at the locations of the weights.

If, in a subsequent test using the same load as the base load or a lesser load, acoustic events similar to the Kaiser effect are heard, this indicates that the structure has been damaged. This damage may have occurred by the corrosion of a wire or reinforcing bar, or because of structural damage from earthquake, collision or the like. The acoustic events are thought to indicate that the weakened structure is undergoing microcracking, even though it would not have undergone microcracking at the loading applied had it not been damaged.

Where there are several sensors, the locations from which the acoustic events were emitted can be found using the location techniques discussed in the Paulson patents cited above or any other known technique for locating the source of acoustic emissions. There are typically many very small acoustic events, and a few larger ones (eg above 0 dB.) Where one or more of the larger events has been recorded by several sensors, the location techniques can be applied to find its origin. Even if the concrete is curved (as in the sidewall of a cylindrical pressure vessel), virtually all of the energy passes through the concrete, and the concrete can be considered for the purpose of finding the origin as though it were a flat sheet. The origin can then be examined using conventional techniques, and the damage can be repaired before it becomes serious.

In a preferred embodiment, tests are repeated periodically, (for example once every 1-6 months) using loads equal to, or less than the base load. These permit the finding of new damage which has occurred since the previous test. In this way, damage can often be detected and corrected before it becomes major enough to threaten the structural integrity of the vessel or structure.

The drawing illustrates an equipment configuration suitable for this embodiment. Pressure vessel 10 is a concrete vessel suitable for containment of radioactive vapour in case of an explosion at a nuclear power plant. It has a cylindrical concrete sidewall 11 and a circular concrete roof 12. It also has a circular concrete floor (not shown). A typical such vessel is for example 10 m. in diameter and the sidewall 11 is 13 m. in height and ½ m. thick. It is designed to operate at an outward pressure from its contents of 5.5 bar (80 pounds per square inch) over atmospheric pressure (5,5 bar, 80 psig), and to be able to withstand considerably higher outward pressures in an emergency.

On sidewall 11 is mounted acoustic sensor 20, which senses acoustic events in a frequency range of 2 KHz to 12 KHz. The acoustic events sensed in this range are primarily acoustic waves which reach the sensor through the concrete wall. Acoustic sensor 20 is connected by cable 21 to recording device 22 so that acoustic waves registering at sensor 20 cause sensor 20 to emit electrical signals which are recorded at recording device 22. Preferably the recording device 22 is a computer which also has the capacity to analyze the signals, by making Fourier transforms thereof to determine their spectral density.

Optionally, additional sensors 23 and 25 are located on the sidewall 11, and are connected to recording device 22 by cables 24 and 26 respectively. Preferably, the sensors used are the same as sensor 20.

To provide a base level for later comparison, the pressure in the containment vessel is raised to 120 psig which is about 8.3 bar over atmospheric pressure, and which is a pressure level unlikely to be encountered in normal operation. If the vessel has not previously been pressurized to this pressure, Kaiser events will occur. If it has been pressurized previously to this level, Kaiser events will not occur in the absence of damage or corrosion. If Kaiser events do occur, they are preferably recorded, and the locations at which they occurred are inspected to see if there was damage.

In subsequent periodic tests, the pressure in the vessel is raised to a test pressure of 120 psig (8.3 bar) or, if preferred, to a lower test pressure which is still in excess of the normal operating pressure of 80 psig. (5.5 bar), such as for example 100 psig. (6.9 bar). When the test pressure is reached, the sensors are monitored for acoustic events in the range 2-12 KHz. It is expected that no acoustic events will be recorded (except possibly ones from extraneous sources, such as passing traffic etc.) If acoustic events are recorded in this range, and are not otherwise explicable, damage or corrosion to the structure is considered likely. Preferably, the origin or origins of the acoustic events are determined, as for example at 40. The sidewall 11 is then examined at location 40 by conventional means to determine whether there is corrosion or damage at that location.

### Continuous monitoring embodiment

In another embodiment of the invention, continuous acoustic monitoring for acoustic events in the 2-12 KHz frequency range, or some frequency range within this selected after consideration of the particular vessel or structure, is carried out. The preferred range is 8-12KHz. Other frequencies can either be discarded or can be recorded for purposes unrelated to this invention Suitable filters can be included if desired to record only those acoustic emissions which are in the frequency range of 2-12 KHz or some smaller frequency range within this range where the structure of vessel is known to exhibit a Kaiser effect.

Where acoustic events occur in the selected range, the recorded signals from such events can be examined later and compared with reference recordings of cracking sounds recorded in previous tests the same structure or similar structures, to see if the newly recorded acoustic events have similar energy spectra and duration, and therefore are likely to have been caused by microcracking. Alternately, the acoustic events can be compared to reference recordings by computer immediately after the events are recorded, and a report can be generated immediately if the events are identified as probable microcracking. This embodiment permits the collecting of microcracking information at the time the microcracking occurs. The origin of the acoustic events can be found by applying known methods of analysis (such as those shown in the Paulson patents discussed) to the output of several sensors, In cases where damage occurs through sudden events (such as a collision or a seismic event such as an earthquake) this embodiment permits location of possibly damaged areas, so that they can be inspected and repaired if necessary.

Preferably, when continuous monitoring is carried out, apparatus such as that shown in Figure 1 is used. Testing is first done by loading the structure, as in the tests discussed above, to determine the frequency spectrum and duration of acoustic events made by microcracking in the structure. Then, recording device 22 is a computer programmed to recognize the acoustic "signatures" of the microcracking events, in terms of frequencies and duration. Events having similar frequencies and duration can be logged for further spectral examination to see if they are in fact microcracking events, or can immediately be used to cause an on-site inspection to be carried out.

As discussed above, it has been found that, when the structure has been damaged, the acoustic events representing microcracking appears at a lower load level than was previously the case. For example, in a containment vessel, corrosion of reinforcing bars may produce weakening of some sections of the vessel. If acoustic monitoring of the entire vessel shows the appearance of microcracking at a lower pressure than was previously the case, the existence of the damage can be discovered, and its location can be determined. Similarly, the appearance of cracking events during a seismic event allows damaged areas to be identified even if the cracking is not visible on the exterior of the vessel. Subsequent pressurization of the vessel can then be carried out to reveal information about the impact of the damage on the ability of the vessel to contain required pressures. Thus small or concealed damage can be located before it has a chance to get worse or cause a failure of the vessel of structure.

### Analysis of the Seriousness of Cracking

In a further embodiment, useable in association with either periodic testing under load or continuous monitoring, the signals showing cracking are examined to assess the seriousness of the cracking. Typically, the signals are a series of individual, discrete, signals. The number of such signals per unit of time is examined. When a structure or vessel is loaded in excess of its previous loading, the Kaiser effect gives rise to signals. It is found that the rate of cracking caused by the Kaiser effect normally diminishes quickly after a given load is achieved. Depending on the structure, the rate of acoustic events (number of acoustic events in the 2-12KHz range per second) diminished by half in an approximately constant period after the greater pressure than previous had been reached. For any structure or vessel, the decrease in rate is approximately constant and characteristic to that vessel. For some vessels, the rate decreases by half every 10 minutes after a new pressure level had been achieved, and for others the rate decreases by half in less than two minutes. Generally, it is expected that the rate will decrease by half in from 15 seconds to 15 minutes, depending on the particular structure or vessel.

Periodic tests of pressure vessels or structures according to the invention show the same pattern where the vessel or structure is loaded to a loading at or below previous loadings, but where there has been minor corrosion damage between tests: i.e., the rate of acoustic events reduces approximately by half every 15 seconds to 15 minutes after a level of loading that gives rise to acoustic emissions is reached and maintained. Therefore, if the rate of diminution of acoustic emissions does not diminish by half in a time period of the order of 15 seconds-15 minutes, then the vessel is not responding as expected and requires immediate attention. A steady rate of the emissions denoting cracking, for example, would indicate progressive damage to the vessel is occurring even during the test. Under such circumstances, the test should be discontinued, even though no structural damage is evident, and steps should immediately be taken to do conventional inspection and possible repair. Similarly, during continuous monitoring, a steady rate of acoustic events denoting cracking indicates that immediate conventional inspection of the vessel or structure is required.

## Claims

1. A method of inspection of concrete structures which comprises:
a) conducting a base test by loading the structure with a base load which is considerably in excess of the load to which the structure is exposed in normal use,
b) removing the base load,
c) subsequently conducting at least one further test by loading the structure with a test load equal to or less than the base load, and exerting loading in approximately the same location as in the base test;
d) noting acoustic events, if any, which are detected at one or more frequencies in the range of 2 KHz to 12 KHz by at least one sensor in contact with the structure while the structure is loaded with the test load;
e) if acoustic events not otherwise explicable are noted, examining the structure by conventional means to determine whether there is damage or corrosion.

2. A method as claimed in claim 1, where the acoustic events are detected at one or more frequencies in the range of 8 KHz to 12 KHz.

3. A method as claimed in claim 1 or claim 2, in which the acoustic events are detected by a plurality of sensors, and the origin of at least some such signals is determined by comparing the emissions detected at each sensor.

4. A method as claimed in claim 1, in which the base test and the subsequent test are conducted by,
(i) positioning at least three acoustic sensors in locations to sense acoustic emissions at a frequency range within the range of 2KHz to 12KHz within the structure,
(ii) monitoring the output of such sensors.

5. A method as claimed in claim 4, in which the step e) of noting of acoustic events not otherwise explicable and examining the structure includes,
(i) if and when such sensors detect acoustic events characteristic of concrete microcracking, determining the probable origin of such acoustic events, and
(ii) inspecting the structure in the vicinity of the probable origin for evidence of damage.

6. A method as claimed in any of claims 1-5, in which, if acoustic events are noted at the test load, then such acoustic events are monitored and a determination is made as to how long it takes for the rate of such events to decline by one-half.

7. A method as claimed in any of claims 1-6, in which the structure is a pressure vessel, and the loading is changed by changing the pressure in the vessel.

8. A method as claimed in claim 4, in which the frequency range is within the range of 8KHz to 12KHz.

9. A method as claimed in claim 5, in which, once such sensors detect acoustic events characteristic of concrete microcracking, the number of acoustic events per second is determined, and whether such number of acoustic events per second is declining by half in a time period of the order of 15 seconds - 15 minutes is determined.

## Patentansprüche

1. Verfahren zur Inspektion von Betonbauwerken, welches beinhaltet:
a) Durchführen eines Grundtests durch Belasten des Bauwerks mit einer Grundlast, die beträchtlich größer ist als die Belastung, der das Bauwerk im normalen Gebrauch ausgesetzt ist,
b) Entfernen der Grundlast,
c) anschließendes Durchführen von wenigstens einem weiteren Test durch Belasten des Bauwerks mit einer Testlast, die gleich der oder kleiner als die Grundlast ist, und Ausüben der Belastung an ungefähr derselben Stelle wie bei dem Grundtest,
d) Feststellen von akustischen Ereignissen, falls vorhanden, die mit einer oder mehreren Frequenzen in dem Bereich von 2 kHz bis 12 kHz durch wenigstens einen Sensor erfasst werden, der mit dem Bauwerk in Kontakt ist, während das Bauwerk mit der Testlast belastet ist;
e) wenn akustische Ereignisse, die anderweitig nicht erklärbar sind, festgestellt werden, Prüfen des Bauwerks durch herkömmliche Maßnahmen, ob Beschädigung oder Korrosion vorhanden ist.

2. Verfahren nach Anspruch 1, wobei die akustischen Ereignisse mit einer oder mehreren Frequenzen in dem Bereich von 8 kHz bis 12 kHz erfasst werden.

3. Verfahren nach Anspruch 1 oder 2, bei welchem die akustischen Ereignisse durch eine Vielzahl von Sensoren erfasst werden und der Ursprung von wenigstens einigen der Signale bestimmt wird durch Vergleichen der von jedem Sensor erfassten Emissionen.

4. Verfahren nach Anspruch 1, bei welchem der Grundtest und der anschließende Test ausgeführt werden durch
(i) Positionieren von wenigstens drei akustischen Sensoren an Orten zum Erfassen von akustischen Emissionen in einem Frequenzbereich innerhalb des Bereiches von 2 kHz bis 12 kHz innerhalb des Bauwerks,
(ii) Überwachen der Ausgangssignale der Sensoren.

5. Verfahren nach Anspruch 4, bei welchem der Schritt e) des Feststellens von akustischen Ereignissen, die anderweitig nicht erklärbar sind, und des Prüfens des Bauwerks beinhaltet
(i) falls und wenn die Sensoren akustische Ereignisse erfassen, die für Mikrorissbildung in Beton charakteristisch sind, Feststellen des wahrscheinlichen Ursprungs der akustischen Ereignisse, und
(ii) Inspizieren des Bauwerks in der Nähe des wahrscheinlichen Ursprungs für das Vorliegen von Beschädigung.

6. Verfahren nach einem der Ansprüche 1 - 5, bei welchem, falls akustische Ereignisse bei der Testlast festgestellt werden, dann diese akustischen Ereignisse überwacht werden und ermittelt wird, wie lange es dauert, bis die Rate von diesen Ereignissen um die Hälfte abgenommen hat.

7. Verfahren nach einem der Ansprüche 1 - 6, bei weichem das Bauwerk ein Druckbehälter ist und die Belastung geändert wird durch Ändern des Druckes in dem Behälter.

8. Verfahren nach Anspruch 4, bei welchem der Frequenzbereich innerhalb des Bereiches von 8 kHz bis 12 kHz ist.

9. Verfahren nach Anspruch 5, bei welchem, nachdem die Sensoren akustische Ereignisse erfasst haben, die für Mikrorissbildung in Beton charakteristisch sind, ermittelt werden die Zahl der akustischen Ereignisse pro Sekunde und ob diese Zahl von akustischen Ereignissen pro Sekunde in einer Zeitspanne in der Größenordnung von 15 Sekunden - 15 Minuten um die Hälfte abnimmt.

## Revendications

1. Procédé d'inspection de structures en béton, comprenant les étapes suivantes :
a) conduite d'un test de base en chargeant la structure avec une charge de base qui dépasse largement la charge à laquelle la structure est soumise en utilisation normale ;
b) retrait de la charge de base ;
c) conduite ultérieure d'au moins un test supplémentaire en chargeant la structure par une charge de test égale ou inférieure à la charge de base, et application de la charge approximativement au même endroit que pour celle du test de base ;
d) rapport, le cas échéant, d'événements acoustiques détectés à une ou plusieurs fréquences situées dans la gamme comprise entre 2 kHz et 12 kHz par au moins un capteur en contact avec la structure alors que la structure est chargée par la charge de test ;
e) si on note des événements acoustiques non explicables autrement, examen de la structure par des moyens conventionnels pour déterminer des dommages ou de la corrosion.

2. Procédé selon la revendication 1, dans lequel les événements acoustiques sont détectés à une ou plusieurs fréquences dans la gamme comprise entre 8 kHz et 12 kHz.

3. Procédé selon la revendication 1 ou la revendication 2, dans lequel les événements acoustiques sont détectés par une pluralité de capteurs, et dans lequel l'origine d'au moins certains de tels signaux est déterminée en comparant les émissions détectées au niveau de chaque capteur.

4. Procédé selon la revendication 1, dans lequel le test de base et le test ultérieur sont effectués selon les étapes suivantes :
(i) positionnement d'au moins trois capteurs acoustiques en certains emplacements pour détecter des émissions de la structure dans une gamme de fréquences comprises entre 2 kHz et 12 kHz ;
(ii) enregistrement de la sortie de tels capteurs.

5. Procédé selon la revendication 4, dans lequel l'étape e) de constatation d'événements acoustiques ne pouvant être expliqués autrement et d'examen de la structure consiste en :
(i) si et lorsque de tels capteurs détectent des événements acoustiques caractéristiques de microfissures du béton, la détermination de l'origine probable de tels événements acoustiques, et
(ii) l'inspection de la structure à proximité de l'origine probable de signes de dommage.

6. Procédé selon l'une quelconque des revendications 1 à 5, dans lequel si des événements acoustiques sont détectés pour la charge de test, de tels événements acoustiques sont alors enregistrés et une détermination est effectuée pour connaître la durée nécessaire au taux de tels événements acoustiques pour diminuer de moitié.

7. Procédé selon l'une quelconque des revendications 1 à 6, dans lequel la structure est une cuve sous pression, et dans lequel la charge est modifiée en changeant la pression dans la cuve.

8. Procédé selon la revendication 4, dans lequel la gamme de fréquences est comprise entre 8 kHz et 12 kHz.

9. Procédé selon la revendication 5, dans lequel une fois que de tels capteurs détectent des événements acoustiques caractéristiques de microfissures du béton, le nombre d'événements acoustiques par seconde est déterminé, et on détermine si un tel nombre d'événements acoustiques par seconde diminue de moitié pendant une période de l'ordre de 15 secondes à 15 minutes.
